# EUROPEAN PATENT APPLICATION

(11) **EP 2 191 796 A1**
(43) Date of publication of application: **02.06.2010**
(21) Application number: 08170186.4
(22) Date of filing: 28.11.2008
(51) Int. Cl.: A61F 5/00

(54) **Artificial sphincter assembly**

(71) Applicant: DEBIOTECH S.A., 1004 Lausanne (CH)
(72) Inventor: Schneeberger, Niklaus, 1004, Lausanne (CH); Neftel, Frédéric, 1005, Lausanne (CH)
(74) Representative: Grosfillier, Philippe

(57) **Abstract**

An implantable artificial sphincter assembly comprising an hydraulically adjustable band (1), a fluid reservoir (2), a bidirectional fluid line (3,4,15,16) connecting said reservoir to said band and pumping means (5) which are adapted to move a fluid along said fluid line (3,4), said pumping means (5) being located on the fluid line (3,4).

## Description

### Field of invention

The present invention relates to implantable artificial sphincters such as gastric bands.

It more precisely relates to artificial sphincters which are hydraulically controlled.

### State of the art

Hydraulically adjustable gastric bands are known since decades. See for instance the following patent documents : EP 1 600 183 A1, EP 1 600 128 A1, EP 1 832 252 A2 or EP 1 967 168 A2.

Those assemblies comprise bidirectional pumping means such as a syringe, linear or rotary actuators, which are connected to the reservoir which itself is in fluid communication with an adjustable gastric band. In order to move the fluid into the gastric band the reservoir has to be pressurized.

This pressure requirement implies the use of a rigid reservoir.

As a separate matter, one of the problems with pump actuated artificial sphincters is that they require a reservoir and a pumping mechanism to be implemented through invasive techniques because of the size and rigid configuration. The size of the reservoir being encapsulated into a rigid housing able to withstand high pressure and protecting the mechanism (such as titanium devices, usually the size of a hockey puck).

### General description of the invention

The present invention offers the opportunity to use a flexible reservoir instead of a rigid one, making therefore possible a direct connection between the artificial sphincter and the reservoir.

One of the purpose of the invention is to provide a pumping device which can be integrated into a gastric band so that it can be introduced into the patient by minimal invasive techniques (such as laparoscopy). For that purpose, a soft reservoir is required, which does not need to be pressurized, as well as a miniaturized pumping device, as both will be described further in the description of this invention.

To this effect the invention refers to an implantable artificial sphincter assembly comprising an hydraulically adjustable band, a fluid reservoir, a fluid line connecting said reservoir to said band and pumping means which are adapted to move a fluid along said fluid line , said pumping means being located on the fluid line.

In order to operate, the hydraulically adjustable band preferably comprises a compartment filled by fluid which is acting as a compressing mean onto the anatomic tube to be restricted.

In one embodiment , the pumping means are a linear peristaltic pump or digital peristaltic pump such as the one disclosed in US 2008/138218.

In another embodiment , the pumping means are a piezo-electric micropump such as the one disclosed in US 5 085 562.

Advantageously the reservoir is flexible and is fixed to the band without compressing effect.

In one preferred embodiment the band and the reservoir have a common rigid wall and distinct flexible walls.

The present invention provides several advantages with respect to state-of-the-art devices. It considerably reduce the risk of contamination which is present with a implantable reservoir which has to be refilled by a syringe to maintain a predefined pressure.

### Detailed description of the invention

The invention is discussed below in a more detailed way with examples illustrated by the following figures :
Figure 1 shows a preferred embodiment of the invention consisting of a gastric band assembly.
Figure 2 is an enlarged view of the assembly of figure 1 showing of a fluid inflate a gastric band.
Figure 3 is similar to figure but differs in that it shows a fluid flowing back to the reservoir.
Figure 4 better shows a pumping element which may be used in the present invention, together with a pressure sensor.

Numerical references used in the figures :
- 1.: Gastric band
- 2.: Reservoir
- 3.: Fluid line , part I
- 4.: Fluid line part II
- 5.: Pumping means
- 6.: Common wall
- 7.: Pressure sensor
- 8.: Pumping chamber
- 9.: Inlet valve
- 10.: Externally controlled valve
- 11.: Antenna
- 12.: Gastric band flexible wall
- 13.: Reservoir flexible wall
- 14.: Flow restrictor
- 15.: Feed segment
- 16.: Return segment

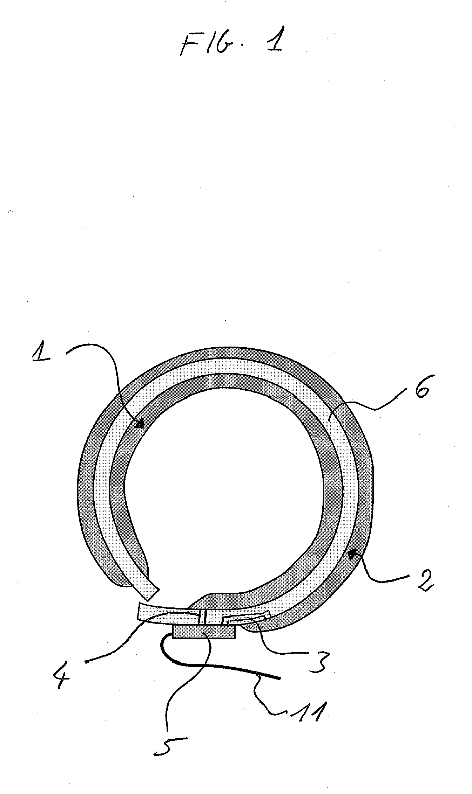

The gastric band assembly illustrated on figure 1 comprises a gastric band 1 and a reservoir which are separated by a common wall 6, preferably a stiff belt.

The gastric band is defined by the common wall 6 and a flexible wall 12.

The reservoir is defined by the common wall 6 and another flexible wall 13.

In figure 1, both gastric band 1 and reservoir 2 are empty, the flexible wall 12, 12 are therefore contacting the common wall.

Figure 1 furthermore shows pumping means 5 which are fixed on the common wall 6.

The gastric band 1 and the reservoir 2 communicate via a fluid line 3, 4 which crosses the common wall 6.

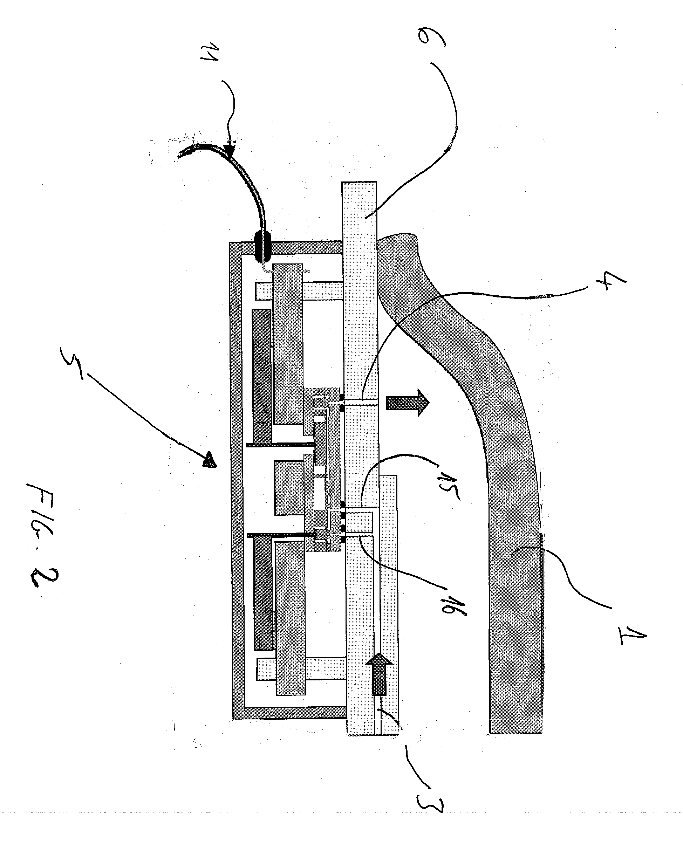

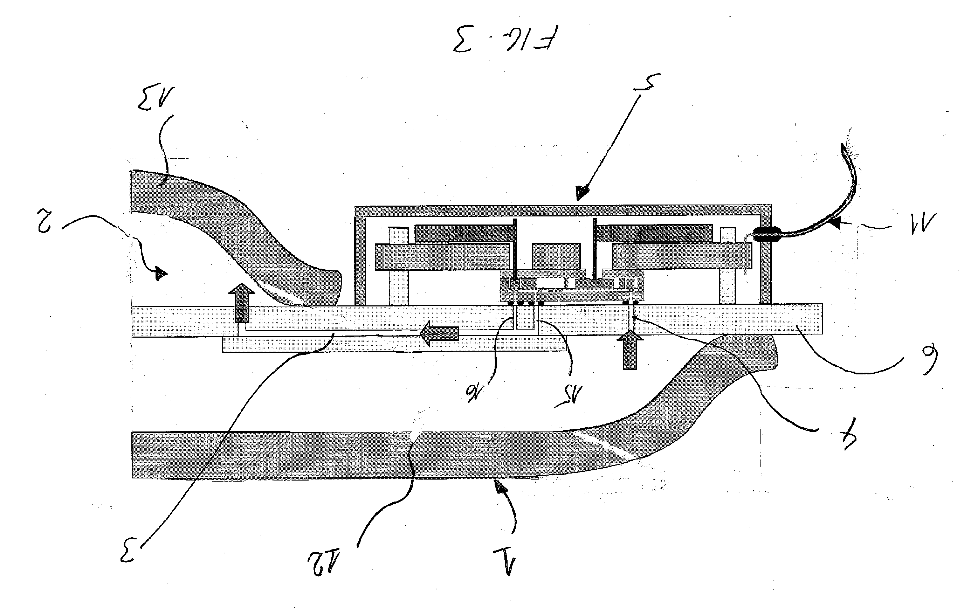

Figure 2 shows a part of the gastric band 1 in a situation when fluid is entering the gastric band internal cavity while figure 3 shows the reverse situation when fluid is flowing back to the reservoir.

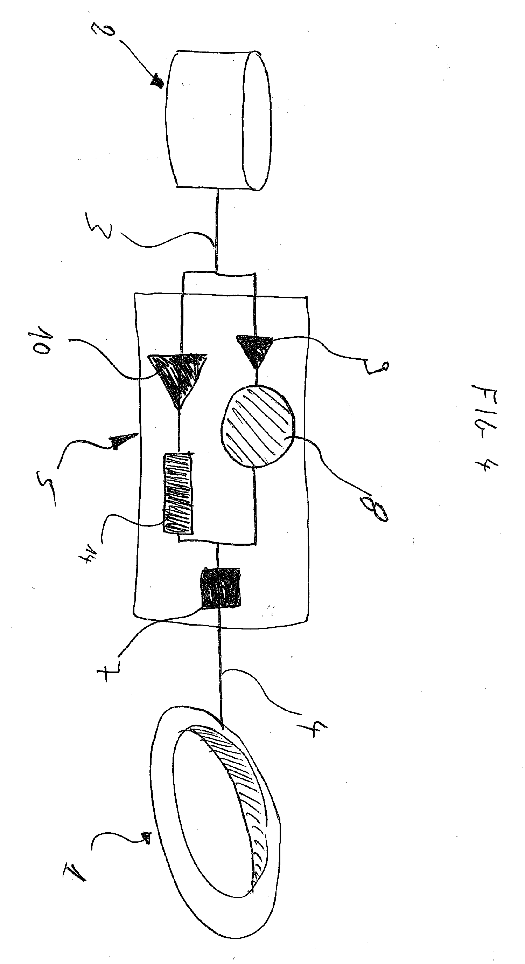

Figure 4 is a simplified view showing the essential elements of a pumping system 5, in particular a pumping chamber 8. A pressure sensor 7 is located between the pumping elements and the gastric band 1. In this case, the assembly preferably includes a feedback loop between the pressure sensor 7 and the pumping means 5 so that the pressure within the gastric band can be permanently adjusted.

The pumping means 5 illustrated on figures 1 to 3 include an antenna. This reception unit allows the actuation of the gastric band 1, i.e. to modify its diameter, from a remote, preferably external, device. Such remote control are disclosed in the prior art.

## Claims

1. An implantable artificial sphincter assembly comprising an hydraulically adjustable band (1), a fluid reservoir (2), a bidirectional fluid line (3,4,15,16) connecting said reservoir to said band (1) and pumping means (5) which are adapted to move a fluid along said fluid line (3,4,15,16), said pumping means (5) being located on the fluid line (3,4,15,16).

2. Assembly according to claim 1 wherein a fluid compartment is included into said band (1) in such a way as to compress the artifical sphincter when it is filled with fluid coming from the reservoir (2).

3. Assembly according to claim 1 or 2 wherein said pumping means (5) are a peristaltic pump type.

4. Assembly according to claim 1 or 2 wherein said pumping means (5) are a piezo-electric micropump type.

5. Assembly according to one of the previous claims wherein said reservoir (2) is flexible and is adapted to not impact by its volume the pressure along the sphincter.

6. Assembly according to claim 5 wherein said reservoir is maintained at a pressure below 100mbar.

7. Assembly according to claim 5 wherein said reservoir (2) is fixed along said band (1).

8. Assembly according to claim 7 wherein said fluid line (3,4,15,16) and said pumping means (5) are fixed to said band (1).

9. Assembly according to claim 7 or 8 wherein said band and said reservoir comprise a common wall (6).

10. Assembly according to claim 9 wherein said common wall (6) is a stiff belt in its length.

11. Assembly according to claim 9 or 10 wherein said fluid line (3,4,15,16) is incorporated in said common wall (6).

12. Assembly according to one of the previous claims comprising a pressure sensor (7) located on the fluid line (3,4,15,16), between said pumping means and said band.

13. Assembly according to claim 12 comprising a feedback loop between said pressure sensor (7) and said pumping means (5).

14. Assembly according to one of the previous claims wherein said pumping means (5) incorporate fluid occlusion means.

15. Assembly according to claim 14 wherein the pumping means can operate bidirectionally.

16. Assembly according to one of the previous claims wherein one part of the fluid line (3,4,15,16) comprises two parallel segments, namely one feed segment (15) and one return segment (16).

17. Assembly according to claim 16 wherein the return segment comprises a valve which can be open to release the pressure in the compartment situated inside the band when required.

18. Assembly according to one of the previous claims wherein said band is a gastric band.

19. Assembly according to one of the previous claims comprising a wireless reception unit adapted to receive actuating signals from a remote device.

20. Assembly according to claim 18 whereby energy is transmitted via an inductive coil to the pumping means.

21. Assembly according to one of the previous claims including an amount of liquid or gel located at least in the reservoir or in the gastric band.

22. Assembly according to claim 21? 17 devoided of any filling port.

23. Assembly according to one of the previous claims comprising a valve adapted to release fluid from the gastric band when the pressure has reached a threshold value.
